# EUROPEAN PATENT SPECIFICATION

(11) **EP 4 402 685 B1**
(45) Date of publication and mention of the grant of the patent: **29.10.2025**
(21) Application number: 22786184.6
(22) Date of filing: 13.09.2022
(51) Int. Cl.: G16H 20/17

(54) **AUTOMATIC TRANSFER OF MEDICAL DEVICE SETTINGS**
AUTOMATISCHE ÜBERTRAGUNG VON EINSTELLUNGEN FÜR MEDIZINISCHE GERÄTE
TRANSFERT AUTOMATIQUE DES PARAMÈTRES DES DISPOSITIFS MÉDICAUX

(30) Priority: 14.09.2021 US 202163244174 P
(43) Date of publication of application: 24.07.2024
(73) Proprietor: CareFusion 303, Inc., San Diego, CA 92130 (US)
(72) Inventor: ABAL, Daniel M., San Diego, CA 92130 (US); BURGESS, Brendan John, San Diego, CA 92130 (US); SUBRAMANIAN, Ramkumar, San Diego, CA 92130 (US)
(74) Representative: Zenz Patentanwälte Partnerschaft mbB
(86) International application number: PCT/US2022/043382
(87) International publication number: WO 2023/043747

(56) References cited:
- EP-A1- 1 881 858
- EP-B1- 1 881 858
- JP-A- 2011 206 571
- US-A1- 2016 256 622
- US-A1- 2021 178 067
- US-B2- 8 185 322

## Description

### BACKGROUND

The present disclosure generally relates to systems and methods of identifying the location of people and equipment, and, in particular, relates to the automatic association of medical equipment with proximate caregivers and patients

When a patient is moved from one ward to another for example from the ICU to GW a pump currently administering a medication to the patient may need to be changed. This results in a change in the available Guardrails and allowable settings for the clinical environment. As a result, certain settings may not be available for the patient's current treatment. The clinician may elect to bypass the dose error reduction software (DERS) and use programming values for another medication, thereby creating the potential for errors. In addition, when changing IV sets to comply with the recommended usage period, a secondary pump and set arrangement is substituted in order to not interrupt the treatment to the patient. This requires the patient's current information and pump settings to be programmed into the secondary changeover set, requiring additional workload and introducing the potential for programming errors.

A commonly followed procedure to ensure the safety of patients being treated in hospitals has been the establishment of the "five rights" of medication administration. These are (1) right patient, (2) right time, (3) right medication, (4) right route of administration, and (5) right dose. Maintaining the required level of attention to ensure that these rights are adhered to and medications and medical substances are properly administered can be a challenge in the hectic environment of a hospital.

Automated processes have been increasingly implemented to reduce the potential for human error. Scanning a barcode that is printed on a wristband worn by a patient, for example, ensures that the patient is correctly identified. This type of process, however, often carries a penalty of increased complexity of the process wherein the nurse must manually scan one or more barcodes, which also increases the amount of time required for the nurse to complete a procedure.

Document EP 1 881 858 A1 discloses a device for controlling multiple infusion pumps, each associated with a specific infusion substance. The pumps administer the substance at a predetermined infusion rate within a defined time frame. The infusion pumps are capable of exchanging control data to synchronize the activation and deactivation of the infusions in a time-coordinated manner.

US 8 185 322 B2 describes a method and system for operating a medical device with or without a cassette. The system allows additional volume to be added to an ongoing infusion without stopping it, while maintaining the infusion parameters.

US 2016 256 622 A1 outlines systems, devices, and methods for delivering substances such as fluids, medications, and drugs using infusion devices with programmable infusion sequences, configuration of infusion steps, and interconnection of multiple devices for synchronized operation during infusion procedures.

### SUMMARY

In order to improve the safety of the patient while switching from a current pump to a new pump, it has been found advantageous to automatically identify the patient and current pump, and automatically identify when a handoff to a new pump is imminent so that the settings can be automatically transferred to the new pump without intervention by the caregiver. In this regard, the five rights of medication administration are safeguarded, even under the most challenging of circumstances, for example within an ICU, emergency room (IR), or operating room (OR).

The disclosed method and system provides for the automatic association of medication administration parameters with a patient and automatic identification of when a handoff between pumps is imminent so that the caregiver may be provide the option to immediately, and without further data entry, transfer the parameters for the patient to the new pump. In some implementations, dose error reduction software (DERS) and Guardrail settings may be set based on the pump's location. For example, a pump currently set up for General Ward can take on the settings for the ICU or Neonatal ward when moved into those locations based on a tracking system that monitors the pump or medical device's location in the hospital. Accordingly, the need for programing the Guardrails and other settings may be eliminated based on the hospital protocols for the individual departments. This reduces the HCP's workload, eliminates potential for entry errors, and provides flexibility in moving medical equipment around the hospital when needed.

According to various implementations of the subject technology, a disclosed system comprises one or more processors configured to: identify that a new infusion device moved into a predetermined area associated with an active infusion device based on signals transmitted from the new and active infusion devices, the active infusion device currently performing an ongoing infusion of a medication to a patient; determine, responsive to identifying the new infusion device moving into the predetermined area with the active infusion device, that an orientation of the new infusion device is indicative of the new infusion device arriving to replace or supplement the active infusion device; responsive to determining the orientation, automatically program the new infusion device with settings currently being used by the active infusion device in the ongoing infusion of the medication to the patient, without user input of the settings; and activate the new infusion device to initiate a new infusion or to continue the ongoing infusion of the medication based on the automatically programmed settings. Other aspects include corresponding methods, apparatuses, and computer program products for implementation of the foregoing features of the disclosed system.

According to various implementations, a machine-implemented method for automatic transferring infusion parameters between infusion devices is disclosed. The method comprises, under the control of one or more electronic devices: identifying that a new infusion device moved into a predetermined area associated with an active infusion device based on signals transmitted from the new and active infusion devices, the active infusion device currently performing an ongoing infusion of a medication to a patient; determining, responsive to identifying the new infusion device moving into the predetermined area with the active infusion device, that an orientation of the new infusion device is indicative of the new infusion device arriving to replace or supplement the active infusion device; responsive to determining the orientation, automatically programming the new infusion device with settings currently being used by the active infusion device in the ongoing infusion of the medication to the patient, without user input of the settings; and activating the new infusion device to initiate a new infusion or to continue the ongoing infusion of the medication based on the automatically programmed setting. Other aspects include corresponding apparatuses, systems, and computer program products for implementation of the foregoing features of the disclosed method.

According to various implementations, also disclosed is a non-transitory machine-readable medium having instructions stored thereon that, when executed by an electronic device, causes the electronic device to perform a method comprising: identifying that a new infusion device moved into a predetermined area associated with an active infusion device based on signals transmitted from the new and active infusion devices, the active infusion device currently performing an ongoing infusion of a medication to a patient; determining, responsive to identifying the new infusion device moving into the predetermined area with the active infusion device, that an orientation of the new infusion device is indicative of the new infusion device arriving to replace or supplement the active infusion device; responsive to determining the orientation, automatically programming the new infusion device with settings currently being used by the active infusion device in the ongoing infusion of the medication to the patient, without user input of the settings; and activating the new infusion device to initiate a new infusion or to continue the ongoing infusion of the medication based on the automatically programmed settings. Other aspects include corresponding methods, apparatuses, and systems for implementation of the foregoing features of the disclosed computer program product.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

The accompanying drawings, which are included to provide further understanding and are incorporated in and constitute a part of this specification, illustrate disclosed embodiments and together with the description serve to explain the principles of the disclosed embodiments. In the drawings:
FIG. 1 depicts an example hospital room where a patient is to receive treatment according to certain aspects of this disclosure.
FIG. 2 is a plan view of the room of FIG. 1 showing the coverage of an example identification system according to certain aspects of this disclosure.
FIG. 3 is an exemplary display of an IV pump requesting selection of one of multiple pumps present in an area according to certain aspects of the disclosure.
FIG. 4 depicts an embodiment wherein a new pump is identified for a parameter handoff based on communications between individual transmitters according to certain aspects of the disclosure.
FIG. 5 is a block diagram of an example system for automatic transferring of patient parameters between infusion devices according to certain aspects of the disclosure.
FIG. 6 is a flowchart depicting an exemplary process for automatic transferring of patient parameters between infusion devices according to certain aspects of the disclosure.
FIG. 7 is a conceptual diagram illustrating an example electronic system for automatic transferring of patient parameters between infusion devices according to certain aspects of the disclosure.

### DETAILED DESCRIPTION

The disclosed methods and system provide for the automatic association of an infusion device near another infusion device within a defined physical space. Once the new infusion device is identified as being near another infusion device currently administering a medication to a patient, the subject technology determines whether the new infusion device has moved in a manner consistent with being associated with the patient and/or or to replace the first infusion device. For example, position information associated with the two infusion devices is determined, including, for example, the position of the two infusion devices in a room, and/or distance and/or angle between the devices. Based on the position information, if the new infusion device moved in a manner consistent with being associated with the patient and/or or to replace the first infusion device then the subject technology automatically transfers current parameters from the infusion device currently administering the medication to the new infusion device.

In the following detailed description, numerous specific details are set forth to provide a full understanding of the present disclosure. It will be apparent, however, to one ordinarily skilled in the art that embodiments of the present disclosure may be practiced without some of the specific details. In other instances, well-known structures and techniques have not been shown in detail so as not to obscure the disclosure.

The method and system disclosed herein are presented in terms of the administration of a medication as an IV fluid using an IV pump to a patient in a hospital. The method and system, however, are equally applicable to other medical settings such as an outpatient clinic and to nonmedical applications where it is desirable to transfer parameters between devices based on a common presence of the devices within a defined physical space. Nothing in this disclosure should be interpreted, unless specifically stated as such, to limit the application of any method or system disclosed herein to a medical or hospital environment.

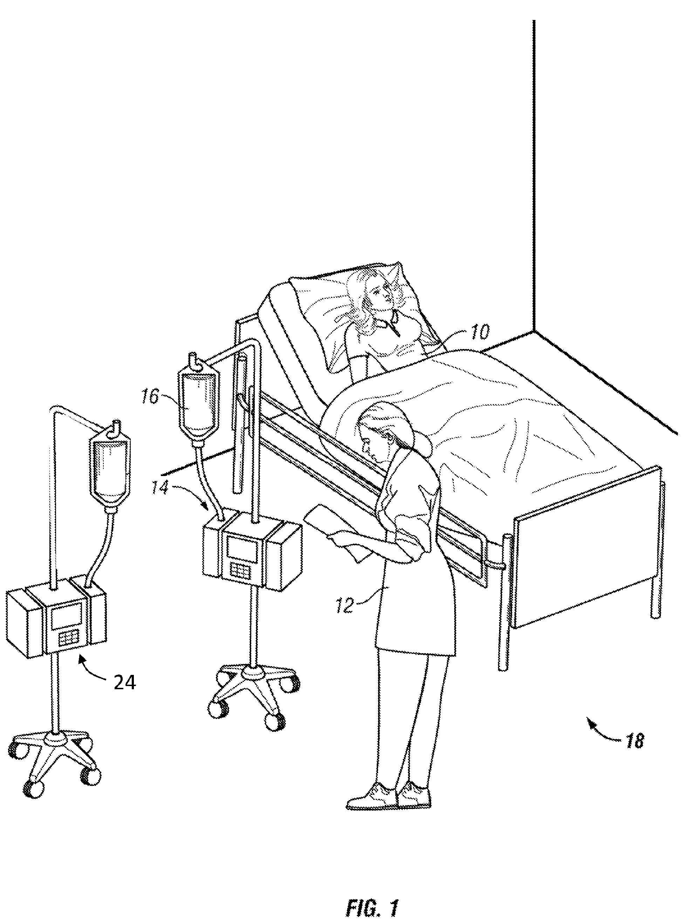

FIG. 1 depicts an example hospital room 18 where a patient 10 is to receive treatment according to certain aspects of this disclosure. In this example, a patient 10 is to receive an infusion of a medication 16 using an IV pump 14, wherein this medication 16 is to be administered by a nurse 12. To ensure the safety of the patient, the nurse 12 must verify that medication 16 has been prescribed for patient 10, ensure that IV pump 14 is configured to deliver the medication 16 at the prescribed rate, and record all of the details of this medication administration in the patient's medication administration record (MAR). Performing these tasks takes time and careful attention and there is always the risk, even with the most conscientious personnel, of making a mistake or forgetting to record something in these manual processes.

A new infusion device 24 is moved into the area, which is already associated with IV pump 14 by way of currently performing an ongoing infusion of a medication 16 to a patient. As will be described further, the new IV pump 24 is identified and responsive to that identifying, a determination is made as to whether an orientation of the IV pump 24 is indicative of the IV pump 24 arriving to replace or supplement the IV pump 14. Responsive to determining the orientation, the system automatically programs the IV pump 24 with settings currently being used by IV pump 14 in the ongoing infusion of the medication 16 to the patient, without user 12 input of the settings, and the IV pump 24 is activated to initiate a new infusion or to continue the ongoing infusion of the medication based on the automatically programmed settings.

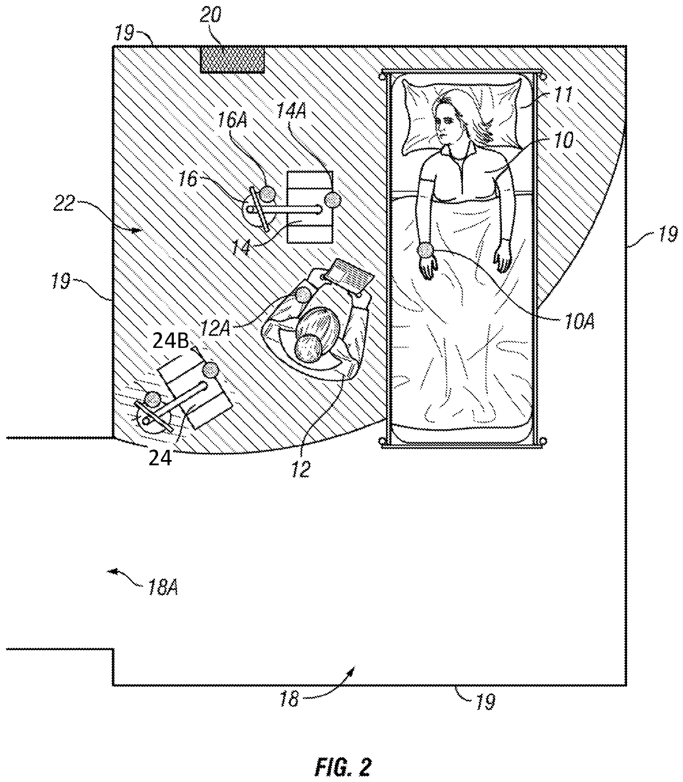

FIG. 2 is a plan view of the room of FIG. 1 showing the coverage of an example identification system according to certain aspects of this disclosure. The identification system identifies infusion devices 14, 24 and tracks the location of the infusion devices 14, 24 in real time. In some implementations, the identification system uses radio frequency transmitters that are attached to or embedded in the devices. For example, each pump may include a wireless transmitter 14A, 24B and the identification system may include at least one transceiver that comprises at least one antenna to receive the wireless signals from the transmitters. In some implementations, as depicted in FIG. 2, the system may include a transceiver 20 that is separate from the infusion devices, for example, mounted on a wall or otherwise positioned within a room of a hospital.

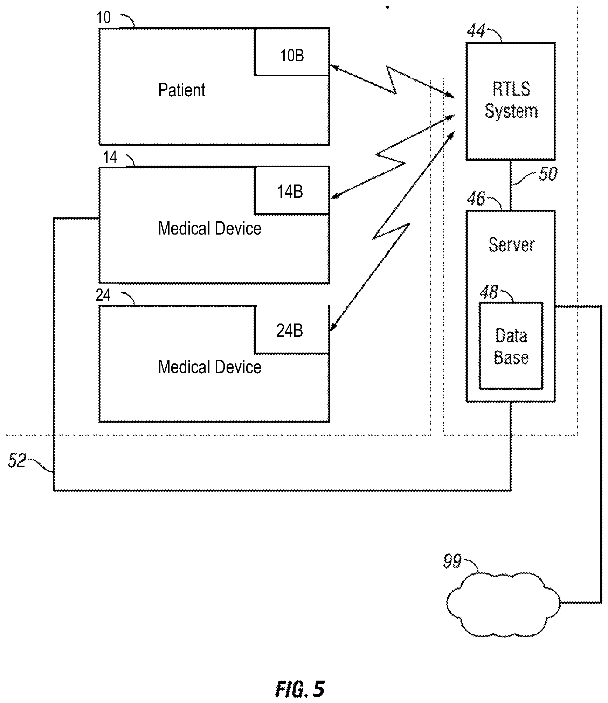

The identification system may be connected to a central controller, e.g. a part of the system 44 of FIG. 5, over, in some cases, a standard communication network such as a wired or wireless Ethernet network 50. The central controller comprises a processor that controls the function of the transceivers and stores, e.g. in database 48 of FIG. 5, the locations of all compatible devices that that are identified within a proximity transceiver (e.g., its range). In some cases, the transceivers communicate wirelessly between each other to form a mesh network that links to the central controller. The range of the antenna of each transceiver may define a physical space, e.g. the physical space 22 of FIG. 2, such that an object is known to be within the physical space when the transceiver is able to receive the wireless signal transmitted by the device (or a compatible transmitter attached to the device). In some implementations, the location of the object within the space can be further determined using the signal strength measured by the transceiver or, if the physical spaces associated with multiple transceivers overlap, receipt of the signal by multiple transceivers.

Each radio transmitter may include a unique identifier that is part of the wireless signal broadcast by the transmitter. For example, a transmitter that is attached to an IV pump 14 may be programmed to either broadcast a code associated with all IV pumps (generic) or broadcast the serial number of that IV pump 14 (specific). Similarly, a transmitter that is embedded in an identification tag of a patient may broadcast the ID number assigned to that patient.

When an infusion device enters a physical space and the signal broadcast by the transmitter is detected by a transceiver associated with that space or a transceiver of another device, the presence of the new device is recorded in the database. When a device is no longer detected by the receiver associated with the physical space in which it was previously detected, or when the device is detected to be in a new physical space, the location information stored in the database may be updated. Thus, the location of all devices are known on a near-real-time basis.

In the example of FIG. 2, a transceiver 20 is mounted on a wall 19 of a patient room 18, wherein the transceiver 20 has a range that defines a physical space 22 (shown as the shaded region in FIG. 2) wherein the transceiver 20 is able to communicate with devices that enter the physical space. The physical space covers a portion of the room 18 that includes the patient bed 11. The range of the transceiver 20 is such that, in this example, it does not extend out the door 18A. The patient 10, currently lying in bed 11, is wearing a radio transmitter 10A that is, in this example, part of his patient identification wristband. The nurse 12 is wearing a radio transmitter 12A as part of her ID badge. The IV pump 14 has a radio transmitter 14A attached to it. According to some implementations, the radio transmitters and/or tags described herein may include an RFID device, Bluetooth device, Ultra Wide Band device, near field communication (NFC) device, or any other device capable of automatically connecting with another device wirelessly and wirelessly communicating with the other device.

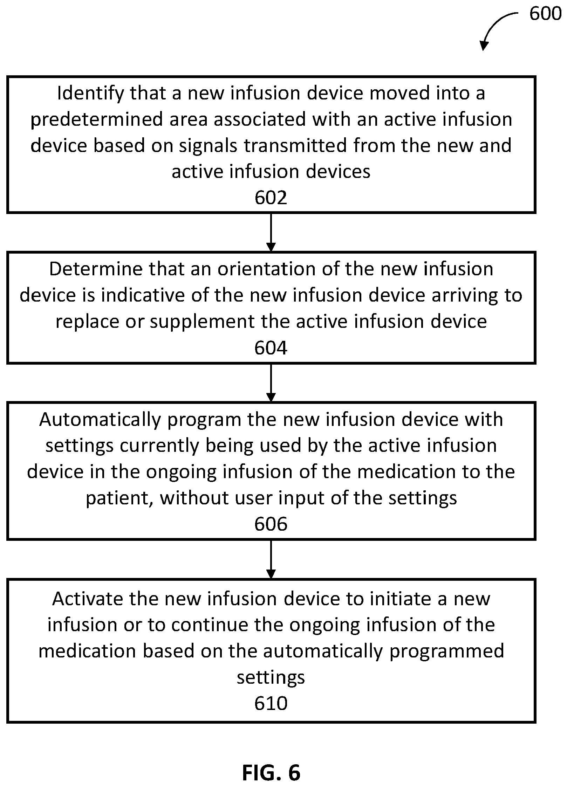

Multiple associations can be made of objects within the defined physical space 22. In this example, the patient was alone in the room 18 prior to the arrival of the nurse 12 with the IV pump 14 and the medication 16. The defined physical space 22 was therefore first associated with patient 10. When the nurse 12 arrives with the IV pump 14, the IV pump 14 is associated with patient 10. When an IV pump 14 has been associated with a patient 10, additional safeguards and automatic actions become possible. For example, parameters may automatically be downloaded to the pump for the patient. When another IV pump 24 enters the physical space 22, it is identified, and the identification system 44 disclosed herein determines whether the device entered into the physical space with the intention to replace the IV pump 14. As will be described further with regard to FIGS. 5 and 6, when this intent is found, the pump settings currently programmed in IV pump 14 and used to administer the medication 16 to the patient may be automatically transferred to a memory of the IV pump 24.

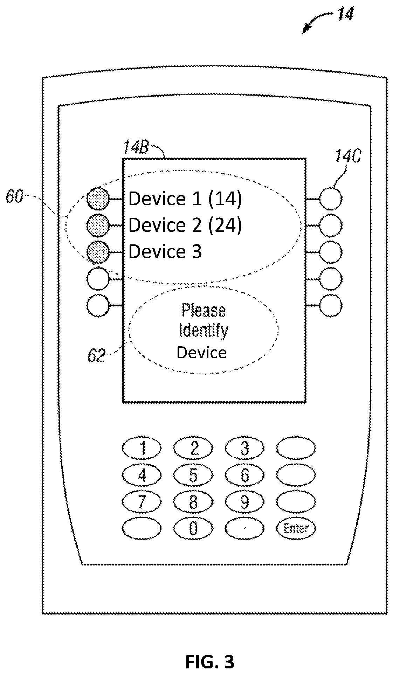

FIG. 3 is an exemplary display of an IV pump 14 requesting selection of one of the infusion devices 12, 24, 26 present in an area according to certain aspects of the disclosure. The IV pump is coupled, in this example, through a wired or wireless communication link, e.g. communication link 52 of FIG. 5, to a server 46 (not shown in FIG. 3) that is coupled to the identification system 44 discussed in more detail in FIG. 5. In this example, a new pump has been identified as being moved into the patient's room, and a prompt is displayed on the pump 14 currently administering a medication. The notification prompts a caregiver to confirm that the new pump is replacing the current pump, and asks whether the caregiver wishes to transfer the pump parameters for the patient to the new infusion device 24.

The example IV pump 14 shown in FIG. 3 comprises a graphic display 14B and a series of buttons 14C. While the buttons are shown on the side of the display, the display 14B may be a touch screen and buttons may be virtual (e.g., designated portions) of the touch screen. For purposes of the depicted example, infusion device 24 may include two or more pump modules, each individually programmable with parameters for a different type of infusion and/or medication.

When the new infusion device 14 enters the same area as infusion device 24, the identification system 44 disclosed herein (e.g., by way of a remote transceiver 20) detects the devices within a proximity of each other and then detects whether (based on factors discussed below) a particular one of the devices has likely moved into the area to replace the current infusion device 24. If multiple devices enter (relatively) at the same time, or there are multiple devices in the room, when another device moves into the room, the currently active device may be caused (e.g., by server 44) to automatically display a notification of the new device and a user interface providing an opportunity to select a device to replace it.

In the depicted example, a list of three devices is automatically presented on the display 14B, as shown within the broken-line oval 60 to prompt the caregiver to choose which pump will be servicing the patient. The display 14B also presents an instructional message as shown within the broken-line oval 62. The pump to be identified is selected by pressing the button 14C that is next to its name. In some implementations, the system will prompt the caregiver to confirm the new device is taking over for the existing device, and that the current parameters used in the current administration should be transferred to the new device. This selection is transmitted back to the server 46 and stored in the database 48. The parameters from the existing device 14 are then transferred (e.g., by the server) to the new device. In certain implementations, the server verifies that the patient has a prescription comprising the medication; and downloads configuration information to the medical device according to the prescription.

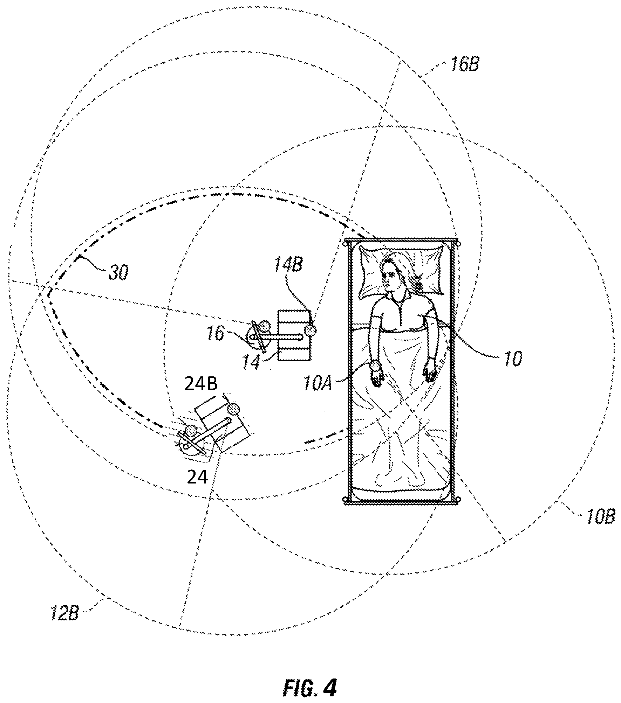

FIG. 4 is a block diagram of an example system for automatic transferring of patient parameters between infusion devices according to certain aspects of the disclosure. In this example, the infusion devices interact among themselves to determine which devices are within close proximity of each other and how they are oriented to determine whether a handoff of parameters between devices is likely. This determination may be based, as will be described further below, on an analysis of positioning information and a determination that certain position information satisfying one or more thresholds. According to some implementations, each transmitter may have a limited range. For example, the transmitter tag 10A worn by patient 10 may have a range indicated by the broken line circle 10B. Similarly, the transmitter 14A on IV pump 14 may have a range of 16B, and the transmitter 24B of IV pump 24 may have a range of 12B. It can be seen that these ranges overlap within the area 30 indicated by the thicker dot-dash line. According to various implementations, the detection and/or determination of orientation of the devices may be performed responsive to the devices being detected within proximity of each other, for example, within the overlap of ranges. For example, when both devices and the patient are within area 30. The orientation information may then be analyzed to determine whether a handoff between the devices is likely.

FIG. 5 is a block diagram of an example configuration of the identification system and the elements to be associated according to certain aspects of the disclosure. In this example, an association system 45 has been created including an identification system 44 and a server 46. According to various implementations, identification system 44 may include a Real Time Locating System (RTLS). System 44 may use BLE (Bluetooth low energy), UWB (Ultra-Wideband) and NFC (Near Field Communications) to identify devices that enter a space, as previously described. In some implementations, system 44 may use ultra wide band for communications between devices. In some implementations, WiFi technology may be utilized to provide tracking of objects by way of tracking connections between respective base stations (e.g., a transceiver 20) and a WiFi capable device.

The server 46 may include a database 48 that contains information about locations of patients and the various medical devices (including infusion devices) and medications prescribed for patients in the hospital, and operating parameters for the medical devices within the hospital.

In this example, the defined physical space 22 contains one patient 10 wearing a tag 10B, a medical device 14 having an attached transmitter 14B, and a medical device 24 having an attached transmitter 24B. This physical space 22 may be a fixed location, such as the interior of a hospital room, or may be a dynamic area surrounding a specific device. For example, a physical space 22 may be defined as centered on the current location of medical device 40, wherein the defined physical space 22 moves with the medical device 40.

While an infusion device is depicted and used to describe the subject technology, the medical device 14, 24 may be any device used to diagnose or treat the patient 10 that is also within the defined physical space 22, such as an electrocardiogram (EKG) measurement system, a ventilator, or a simple ear thermometer. The identification system 44 communicates with server 46 over a data link 50 such as an Ethernet network. Server 46 is also connected, in this example directly to medical device(s) 14, 24 over a data link 52, which may be the same Ethernet network as data link 50 or a separate wired or wireless network or direct connection allowing server 46 to communicate with medical device 40. Server 46 can, for example, download operational parameters from database 48 to medical device 40 and receive information from medical device 14 regarding medical devices that are in proximity to medical device 14 when certain actions or events occurred. Server 46 is also connected to other systems through an internal and/or external network shown as a "cloud" 99. The other systems may include the hospital's Admit/Discharge/Transfer (ADT) system, a MAR system, a billing system, and portable devices carried by the doctors or nurses. The connection of the identification system 44 to the server 46 may be over the Internet or other networks, for example.

FIG. 6 is a flowchart depicting an exemplary process for automatic transferring of patient parameters between infusion devices according to certain aspects of the disclosure. For explanatory purposes, the various blocks of example process 200 are described herein with reference to FIGS. 1-5, and the components and/or processes described herein. The one or more of the blocks of process 600 may be implemented, for example, by one or more computing devices including, for example, medical device 12. In some implementations, one or more of the blocks may be implemented based on one or more machine learning algorithms. In some implementations, one or more of the blocks may be implemented apart from other blocks, and by one or more different processors or devices. Further for explanatory purposes, the blocks of example process 600 are described as occurring in serial, or linearly. However, multiple blocks of example process 600 may occur in parallel. In addition, the blocks of example process 600 need not be performed in the order shown and/or one or more of the blocks of example process 600 need not be performed.

In the depicted example, the disclosed identification system identifies that a new infusion device moved into a predetermined area associated with an active infusion device based on signals transmitted from the new and active infusion devices (602). According to various implementations, when this step occurs, the active infusion device is currently performing an ongoing infusion of a medication to a patient.

In some implementations, the predetermined area is at least part of a new care area different than a care area in which the new infusion device was last programmed. Each care area may have different settings and parameters for programming infusion devices within the care area. In this regard, each care area may require hard or soft limits (e.g., infusion "Guardrails") on one or more infusion settings used during infusions in the care area. These limits set maximum thresholds at which an infusion parameter (e.g., rate, dose, etc.) may be set before an action is taken (e.g., an alarm, terminating the infusion, etc.). Soft limits generally may only activate alarms or require confirmation before proceeding, while hard limits may be so restrictive that the parameter may never be allowed to exceed it or, in some cases, require overriding by an caregiver with elevated access privileges. In some implementations, when a new care area is determined, the identification system will determine infusion pump parameters associated with the new care area and automatically program the pump with the care area parameters. In this regard, the infusion device may be programmed with hard or soft limits assigned to the care area.

Responsive to identifying the new infusion device moving into the predetermined area with the active infusion device, the identification system determines that an orientation of the new infusion device is indicative of the new infusion device arriving to replace or supplement the active infusion device (604). In some implementations, determining that the orientation of the new infusion pump is indicative of the new infusion device arriving to replace or supplement the active infusion device includes detecting a distance between the new infusion pump and the active infusion device, and a motion of the new infusion device relative to the active infusion device. The motion may include, for example, a vector including direction of movement, speed or velocity, and/or an angular momentum. Database 48 may include predetermined patterns of movement that are representative of a new device being moved into position to replace or supplement another device. In some implementations, the patterns may be based on training data collected over a period of time. For example, a respective device may record its own movement and upload that movement to the server 46 when the device is activated in place of another device. The server 46, being connected to both devices, may make the determination of when one device replaces another based on activation and deactivation of the respective devices within a certain amount of time and the new device servicing the same patient and/or with the same medication. In this regard, the distance and motion is matched to a predetermined pattern associated with an intent to replace or supplement one infusion device with another, and the new infusion device is automatically programmed further responsive to matching the distance and motion.

Responsive to determining the orientation of the new infusion device is indicative of the new infusion device arriving to replace or supplement the active infusion device, the identification system automatically programs the new infusion device with settings currently being used by the active infusion device in the ongoing infusion of the medication to the patient, without user input of the settings (606). With reference to FIG. 5, the currently active medical device 14 may periodically communicate with server 46 (e.g., via network connection 52). During this communication, the medical device 14 may record a copy of its current settings used in the ongoing administration of the medication to the patient. In some implementations, the current settings may be uploaded on detection of the new medical device 24 replacing the current medical device 14. The server 46 may then download the settings directly to the new medical device 24.

In some implementations, a user interface will be automatically provided for display on the new infusion device responsive to matching the distance and motion to the predetermined pattern. The user interface may be prepopulated with a patient identifier for the patient and prompting for confirmation to program the new infusion device with parameters for the patient. Having already obtained and associated the patient with the currently active infusion device, the identification system 44 determines that the currently active infusion device is being replaced with the new infusion pump and forwards identification information for the patient to the new infusion device. In this manner, the caregiver does not have to enter patient information into the device to start the infusion, saving time and resources. When the distance and motion are not matched, the user interface may be accessible to the caregiver but may not be prepopulated with the patient identifier and does not prompt for confirmation to program the new infusion device with parameters for the patient.

In some implementations, a third infusion device may be identified as being in the predetermined area, the third infusion device also performing an ongoing infusion of a medication to a respective patient. The third infusion device may be, for example, a second pump module attached to the same control unit as the infusion device 14. Accordingly, matching the distance and motion to a predetermined pattern associated with an intent to replace or supplement the infusion device 14 may include determining the distance and motion is more aligned with replacing or supplementing the active infusion device than the third infusion device.

According to various implementations, the settings and other parameters of the new infusion device may be compared to parameter restrictions for the new care area. The identification system and/or the new infusion pump may determine that one or more parameters associated with the new infusion device are restricted from being used in the new care area. If restricted, a request to confirm an override of the restriction on the restricted parameters may be provided for display on the new infusion device. A confirmation to override the restriction may then be received (e.g., via the display and/or input of the new (or active) infusion device) from a caregiver. In some implementations, the caregiver may be required to scan an authorized badge before the confirmation can be made. Once the confirmation is received, the new infusion device may be programmed to administer the medication in the new care area according to the one or more parameters without the restriction.

In some implementations, the new infusion device may be automatically programmed with the infusion pump parameters associated with the new care area responsive to determining the orientation. In some implementations, the infusion pump parameters include one or more hard or soft limits on one or more infusion settings corresponding to the settings currently being used by the active infusion device in the ongoing infusion of the medication to the patient. In some implementations, the identification system first determines that the new infusion device is authorized for the new care area before automatically programming the new infusion device.

The new infusion device is then activated to initiate a new infusion or to continue the ongoing infusion of the medication based on the automatically programmed settings (610). In some implementations, the identification system may perform verifications on additional information received at the new infusion device to ensure that the medication is being provided to the right patient, and then send the new infusion device an instruction that it may begin the infusion. In this regard, the infusion device may prompt the caregiver to confirm the instruction or to begin the infusion.

Many of the above-described example process 600, and related features and applications, may also be implemented as software processes that are specified as a set of instructions recorded on a computer readable storage medium (also referred to as computer readable medium), and may be executed automatically (e.g., without user intervention). When these instructions are executed by one or more processing unit(s) (e.g., one or more processors, cores of processors, or other processing units), they cause the processing unit(s) to perform the actions indicated in the instructions. Examples of computer readable media include, but are not limited to, CD-ROMs, flash drives, RAM chips, hard drives, EPROMs, etc. The computer readable media does not include carrier waves and electronic signals passing wirelessly or over wired connections.

The term "software" is meant to include, where appropriate, firmware residing in read-only memory or applications stored in magnetic storage, which can be read into memory for processing by a processor. Also, in some implementations, multiple software aspects of the subject disclosure can be implemented as sub-parts of a larger program while remaining distinct software aspects of the subject disclosure. In some implementations, multiple software aspects can also be implemented as separate programs. Finally, any combination of separate programs that together implement a software aspect described here is within the scope of the subject disclosure. In some implementations, the software programs, when installed to operate on one or more electronic systems, define one or more specific machine implementations that execute and perform the operations of the software programs.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

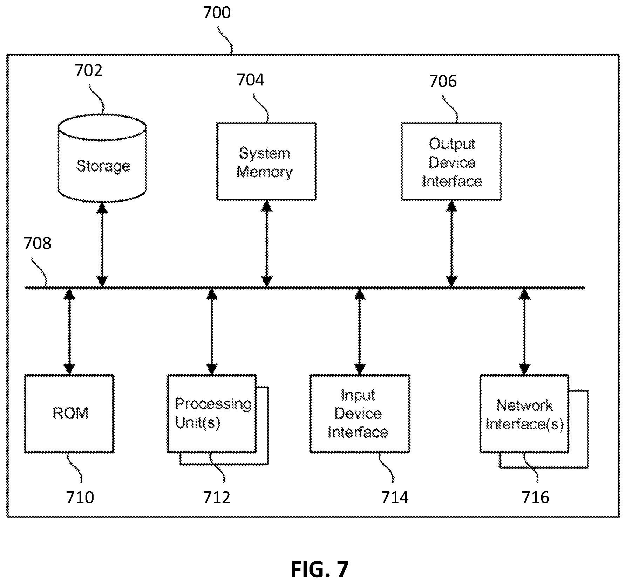

FIG. 7 is a conceptual diagram illustrating an example electronic system 700 for automatic transferring of patient parameters between infusion devices according to certain aspects of the disclosure. Electronic system 700 may be a computing device for execution of software associated with one or more portions or steps of method 700, or components and methods provided by FIGS. 1-6, including but not limited to computing hardware within patient care device 14, 24, system 44, server 46, and/or any computing devices or associated terminals disclosed herein. In this regard, electronic system 700 may be a personal computer or a mobile device such as a smartphone, tablet computer, laptop, PDA, an augmented reality device, a wearable such as a watch or band or glasses, or combination thereof, or other touch screen or television with one or more processors embedded therein or coupled thereto, or any other sort of computer-related electronic device having network connectivity.

Electronic system 700 may include various types of computer readable media and interfaces for various other types of computer readable media. In the depicted example, electronic system 700 includes a bus 708, processing unit(s) 712, a system memory 704, a read-only memory (ROM) 710, a permanent storage device 702, an input device interface 714, an output device interface 706, and one or more network interfaces 716. In some implementations, electronic system 700 may include or be integrated with other computing devices or circuitry for operation of the various components and methods previously described.

Bus 708 collectively represents all system, peripheral, and chipset buses that communicatively connect the numerous internal devices of electronic system 700. For instance, bus 408 communicatively connects processing unit(s) 712 with ROM 710, system memory 704, and permanent storage device 702.

From these various memory units, processing unit(s) 712 retrieves instructions to execute and data to process in order to execute the processes of the subject disclosure. The processing unit(s) can be a single processor or a multi-core processor in different implementations.

ROM 710 stores static data and instructions that are needed by processing unit(s) 712 and other modules of the electronic system. Permanent storage device 702, on the other hand, is a read-and-write memory device. This device is a non-volatile memory unit that stores instructions and data even when electronic system 700 is off. Some implementations of the subject disclosure use a mass-storage device (such as a magnetic or optical disk and its corresponding disk drive) as permanent storage device 702.

Other implementations use a removable storage device (such as a floppy disk, flash drive, and its corresponding disk drive) as permanent storage device 702. Like permanent storage device 702, system memory 704 is a read-and-write memory device. However, unlike storage device 702, system memory 704 is a volatile read-and-write memory, such a random access memory. System memory 704 stores some of the instructions and data that the processor needs at runtime. In some implementations, the processes of the subject disclosure are stored in system memory 704, permanent storage device 702, and/or ROM 710. From these various memory units, processing unit(s) 712 retrieves instructions to execute and data to process in order to execute the processes of some implementations.

Bus 708 also connects to input and output device interfaces 714 and 706. Input device interface 714 enables the user to communicate information and select commands to the electronic system. Input devices used with input device interface 714 include, e.g., alphanumeric keyboards and pointing devices (also called "cursor control devices"). Output device interfaces 706 enables, e.g., the display of images generated by the electronic system 700. Output devices used with output device interface 706 include, e.g., printers and display devices, such as cathode ray tubes (CRT) or liquid crystal displays (LCD). Some implementations include devices such as a touchscreen that functions as both input and output devices.

Also, as shown in FIG. 7, bus 708 also couples electronic system 700 to a network (not shown) through network interfaces 716. Network interfaces 716 may include, e.g., a wireless access point (e.g., Bluetooth or WiFi) or radio circuitry for connecting to a wireless access point. Network interfaces 716 may also include hardware (e.g., Ethernet hardware) for connecting the computer to a part of a network of computers such as a local area network ("LAN"), a wide area network ("WAN"), wireless LAN, or an Intranet, or a network of networks, such as the Internet. Any or all components of electronic system 700 can be used in conjunction with the subject disclosure.

These functions described above can be implemented in computer software, firmware or hardware. The techniques can be implemented using one or more computer program products. Programmable processors and computers can be included in or packaged as mobile devices. The processes and logic flows can be performed by one or more programmable processors and by one or more programmable logic circuitry. General and special purpose computing devices and storage devices can be interconnected through communication networks.

Some implementations include electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra density optical discs, any other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field programmable gate arrays (FPGAs). In some implementations, such integrated circuits execute instructions that are stored on the circuit itself.

As used in this specification and any claims of this application, the terms "computer", "server", "processor", and "memory" all refer to electronic or other technological devices. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; e.g., feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; e.g., by sending web pages to a web browser on a user's client device in response to requests received from the web browser.

Embodiments of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include clients and servers. A client and server are generally remote from each other and may interact through a communication network. The relationship of client and server arises by virtue of computer programs running on the respective computers and having a client-server relationship to each other. In some embodiments, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software depends upon the particular application and design constraints imposed on the overall system. The described functionality may be implemented in varying ways for each particular application. Various components and blocks may be arranged differently (e.g., arranged in a different order, or partitioned in a different way) all without departing from the scope of the subject technology.

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

### Illustration of Subject Technology as Clauses:

Various examples of aspects of the disclosure are described as numbered clauses (1, 2, 3, etc.) for convenience. These are provided as examples, and do not limit the subject technology. Identifications of the figures and reference numbers are provided below merely as examples and for illustrative purposes, and the clauses are not limited by those identification.

### Further Consideration:

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention described herein.

The predicate words "configured to", "operable to", and "programmed to" do not imply any particular tangible or intangible modification of a subject, but, rather, are intended to be used interchangeably. For example, a processor configured to monitor and control an operation or a component may also mean the processor being programmed to monitor and control the operation or the processor being operable to monitor and control the operation. Likewise, a processor configured to execute code can be construed as a processor programmed to execute code or operable to execute code.

The term automatic, as used herein, may include performance by a computer or machine without user intervention; for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such as an "embodiment" may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

As used herein a "user interface" (also referred to as an interactive user interface, a graphical user interface or a UI) may refer to a network based interface including data fields and/or other control elements for receiving input signals or providing electronic information and/or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH^{™}, JAVA^{™}, .NET^{™}, C, C++, web services, or rich site summary (RSS). In some embodiments, a UI may be included in a stand-alone client (for example, thick client, fat client) configured to communicate (e.g., send or receive data) in accordance with one or more of the aspects described. The communication may be to or from a medical device or server in communication therewith.

As used herein, the terms "determine" or "determining" encompass a wide variety of actions. For example, "determining" may include calculating, computing, processing, deriving, generating, obtaining, looking up (e.g., looking up in a table, a database or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like. "Providing" may also include encoding, decoding, encrypting, decrypting, validating, verifying, and the like via a hardware element.

As used herein, the term "message" encompasses a wide variety of formats for communicating (e.g., transmitting or receiving) information. A message may include a machine-readable aggregation of information such as an XML document, fixed field message, comma separated message, JSON, a custom protocol, or the like. A message may, in some implementations, include a signal utilized to transmit one or more representations of the information. While recited in the singular, it will be understood that a message may be composed, transmitted, stored, received, etc. in multiple parts.

As used herein, the term "selectively" or "selective" may encompass a wide variety of actions. For example, a "selective" process may include determining one option from multiple options. A "selective" process may include one or more of: dynamically determined inputs, preconfigured inputs, or user-initiated inputs for making the determination. In some implementations, an n-input switch may be included to provide selective functionality where n is the number of inputs used to make the selection.

As user herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more objects, data sets, information and/or the like so to appear to be the same or equal. Correspondence may be assessed using one or more of a threshold, a value range, fuzzy logic, pattern matching, a machine learning assessment model, or combinations thereof.

**In** any embodiment, data generated or detected can be forwarded to a "remote" device or location, where "remote," means a location or device other than the location or device at which the program is executed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like.

## Claims

1. A system comprising:
one or more processors configured to:
identify that a new infusion device moved into a predetermined area associated with an active infusion device based on signals transmitted between the new and active infusion devices, the active infusion device currently performing an ongoing infusion of a medication to a patient;
determine, responsive to identifying the new infusion device moving into the predetermined area with the active infusion device, that an orientation of the new infusion device is indicative of the new infusion device arriving to replace or supplement the active infusion device;
responsive to determining the orientation, automatically program the new infusion device with settings currently being used by the active infusion device in the ongoing infusion of the medication to the patient, without user input of the settings; and
activate the new infusion device to initiate a new infusion or to continue the ongoing infusion of the medication based on the automatically programmed settings.

2. The system of Claim 1, wherein the predetermined area is at least part of a new care area different than a care area in which the new infusion device was last programmed, wherein the one or more processors are further configured to:
identify the new care area for the new infusion device;
determine infusion pump parameters associated with the new care area; and
automatically program the new infusion device with the infusion pump parameters associated with the new care area responsive to determining the orientation.

3. The system of Claim 2, wherein the infusion pump parameters include one or more hard or soft limits on one or more infusion settings corresponding to the settings currently being used by the active infusion device in the ongoing infusion of the medication to the patient.

4. The system of Claim 2 or Claim 3, wherein the one or more processors are further configured to:
determine that the new infusion device is authorized for the new care area before automatically programming the new infusion device.

5. The system of any one of Claims 2 through 4, wherein the one or more processors are further configured to:
determine that one or more parameters associated with the new infusion device are restricted from being used in the new care area; and
providing, for display on the new infusion device, a request to confirm override of on the restriction;
receiving a confirmation to override the restriction; and
programming, responsive to receiving the confirmation, the new infusion device administer the medication in the new care area according to the one or more parameters without the restriction.

6. The system of any one of Claims 1 through 5, wherein determining that the orientation of the new infusion device is indicative of the new infusion device arriving to replace or supplement the active infusion device comprises:
detecting a distance between the new infusion device and the active infusion device, and a motion of the new infusion device relative to the active infusion device; and
matching the distance and motion to a predetermined pattern associated with an intent to replace or supplement one infusion device with another,
wherein the new infusion device is automatically programmed further responsive to matching the distance and motion.

7. The system of Claim 6, wherein the one or more processors are further configured to:
identify a third infusion device in the predetermined area, the third infusion device currently performing an ongoing infusion of a medication to a respective patient,
wherein matching the distance and motion comprises:
determining the distance and motion is more aligned with replacing or supplementing the active infusion device than the third infusion device.

8. The system of Claim 6 or Claim 7, wherein the one or more processors are further configured to:
provide, for display on a display of the new infusion device, a user interface responsive to matching the distance and motion to the predetermined pattern, the user interface being prepopulated with a patient identifier for the patient and prompting for confirmation to program the new infusion device with parameters for the patient, wherein, when the distance and motion are not matched, the user interface is accessible but is not prepopulated with the patient identifier and does not prompt for confirmation to program the new infusion device with parameters for the patient.

9. The system of any one of Claims 1 through 8, further comprising the new infusion device and the active infusion device, wherein the signals are transmitted between the new and active infusion devices, and wherein the settings currently being used by the active infusion device in the ongoing infusion of the medication to the patient are automatically transferred from a memory of the active infusion device to a memory of the new infusion device.

10. The system of any one of Claims 1 through 9, wherein the one or more processors comprise a server located remote from the new and active infusion devices, the system further comprising:
a locator device configured to identify the new and active infusion devices in the predetermined area and configured to determine the orientation of the new infusion device.

11. A non-transitory machine-readable medium having instructions stored thereon that, when executed by an electronic device, causes the electronic device to perform a method comprising:
identifying that a new infusion device moved into a predetermined area associated with an active infusion device based on signals transmitted between the new and active infusion devices, the active infusion device currently performing an ongoing infusion of a medication to a patient;
determining, responsive to identifying the new infusion device moving into the predetermined area with the active infusion device, that an orientation of the new infusion device is indicative of the new infusion device arriving to replace or supplement the active infusion device;
responsive to determining the orientation, automatically programming the new infusion device with settings currently being used by the active infusion device in the ongoing infusion of the medication to the patient, without user input of the settings; and
activating the new infusion device to initiate a new infusion or to continue the ongoing infusion of the medication based on the automatically programmed settings.

12. The non-transitory machine-readable medium of Claim 11, wherein the predetermined area is at least part of a new care area different than a care area in which the new infusion device was last programmed, wherein the method further comprises:
identify the new care area for the new infusion device;
determine infusion pump parameters associated with the new care area, wherein the infusion pump parameters include one or more hard or soft limits on one or more infusion settings corresponding to the settings currently being used by the active infusion device in the ongoing infusion of the medication to the patient; and
automatically program the new infusion device with the infusion pump parameters associated with the new care area responsive to determining the orientation.

## Patentansprüche

1. System, umfassend:
einen oder mehrere Prozessoren, die konfiguriert sind zum:
Erkennen, dass ein neues Infusionsgerät in einen vordefinierten Bereich bewegt wurde, der einem aktiven Infusionsgerät zugeordnet ist, basierend auf Signalen, die zwischen dem neuen und dem aktiven Infusionsgerät übertragen werden, wobei das aktive Infusionsgerät aktuell eine laufende Infusion eines Medikaments an einen Patienten durchführt;
Bestimmen, responsiv auf das Erkennen des neuen Infusionsgeräts im vordefinierten Bereich mit dem aktiven Infusionsgerät, dass eine Orientierung des neuen Infusionsgeräts darauf hinweist, dass das neue Infusionsgerät zum Ersetzen oder Ergänzen des aktiven Infusionsgeräts eingesetzt wird;
responsiv auf das Bestimmen der Orientierung, automatisch das neue Infusionsgerät mit Einstellungen zu programmieren, die aktuell von dem aktiven Infusionsgerät bei der laufenden Infusion des Medikaments an den Patienten verwendet werden, ohne Benutzereingabe der Einstellungen; und
Aktivieren des neuen Infusionsgeräts zum Initiieren einer neuen Infusion oder zum Fortsetzen der laufenden Infusion des Medikaments basierend auf den automatisch programmierten Einstellungen.

2. System nach Anspruch 1, wobei der vordefinierte Bereich mindestens einen Teil eines neuen Versorgungsbereichs umfasst, der sich von einem Versorgungsbereich unterscheidet, in dem das neue Infusionsgerät zuletzt programmiert wurde, wobei der eine oder die mehreren Prozessoren ferner konfiguriert sind zum:
Identifizieren des neuen Versorgungsbereichs für das neue Infusionsgerät;
Bestimmen von Infusionspumpenparametern, die dem neuen Versorgungsbereich zugeordnet sind; und
automatisches Programmieren des neuen Infusionsgeräts mit den Infusionspumpenparametern, die dem neuen Versorgungsbereich zugeordnet sind, responsiv auf das Bestimmen der Orientierung.

3. System nach Anspruch 2, wobei die Infusionspumpenparameter einen oder mehrere harte oder weiche Grenzwerte für eine oder mehrere Infusionseinstellungen umfassen, die den Einstellungen entsprechen, die aktuell von dem aktiven Infusionsgerät bei der laufenden Infusion des Medikaments an den Patienten verwendet werden.

4. System nach Anspruch 2 oder 3, wobei der eine oder die mehreren Prozessoren ferner konfiguriert sind zum:
Bestimmen, dass das neue Infusionsgerät für den neuen Versorgungsbereich autorisiert ist, vor dem automatischen Programmieren des neuen Infusionsgeräts.

5. System nach einem der Ansprüche 2 bis 4, wobei der eine oder die mehreren Prozessoren ferner konfiguriert sind zum:
Bestimmen, dass einer oder mehrere Parameter, die dem neuen Infusionsgerät zugeordnet sind, für die Verwendung im neuen Versorgungsbereich eingeschränkt sind;
Bereitstellen einer Aufforderung zur Bestätigung der Aufhebung der Einschränkung zur Anzeige auf dem neuen Infusionsgerät;
Empfangen einer Bestätigung zur Aufhebung der Einschränkung; und
Programmieren, responsiv auf das Empfangen der Bestätigung, des neuen Infusionsgeräts zur Verabreichung des Medikaments im neuen Versorgungsbereich gemäß dem einen oder den mehreren Parametern ohne die Einschränkung.

6. System nach einem der Ansprüche 1 bis 5, wobei das Bestimmen, dass die Orientierung des neuen Infusionsgeräts darauf hinweist, dass das neue Infusionsgerät zum Ersetzen oder Ergänzen des aktiven Infusionsgeräts eingesetzt wird, umfasst:
Erfassen eines Abstands zwischen dem neuen Infusionsgerät und dem aktiven Infusionsgerät sowie einer Bewegung des neuen Infusionsgeräts relativ zum aktiven Infusionsgerät; und
Abgleichen des Abstands und der Bewegung mit einem vordefinierten Muster, das einer Absicht zum Ersetzen oder Ergänzen eines Infusionsgeräts durch ein anderes zugeordnet ist,
wobei das neue Infusionsgerät zusätzlich responsiv auf das Abgleichen des Abstands und der Bewegung automatisch programmiert wird.

7. System nach Anspruch 6, wobei der eine oder die mehreren Prozessoren ferner konfiguriert sind zum:
Identifizieren eines dritten Infusionsgeräts im vordefinierten Bereich, wobei das dritte Infusionsgerät aktuell eine laufende Infusion eines Medikaments an einen jeweiligen Patienten durchführt,
wobei das Abgleichen des Abstands und der Bewegung umfasst:
Bestimmen, dass der Abstand und die Bewegung stärker mit dem Ersetzen oder Ergänzen des aktiven Infusionsgeräts als des dritten Infusionsgeräts übereinstimmen.

8. System nach Anspruch 6 oder 7, wobei der eine oder die mehreren Prozessoren ferner konfiguriert sind zum:
Bereitstellen einer Benutzerschnittstelle zur Anzeige auf einem Display des neuen Infusionsgeräts, responsiv auf das Abgleichen des Abstands und der Bewegung mit dem vordefinierten Muster, wobei die Benutzerschnittstelle mit einer Patientenkennung für den Patienten vorbelegt ist und eine Bestätigungsaufforderung zum Programmieren des neuen Infusionsgeräts mit Parametern für den Patienten enthält, wobei bei nicht übereinstimmendem Abstand und Bewegung die Benutzerschnittstelle zugänglich, jedoch nicht mit der Patientenkennung vorbelegt ist und keine Bestätigungsaufforderung zum Programmieren des neuen Infusionsgeräts mit Parametern für den Patienten enthält.

9. System nach einem der Ansprüche 1 bis 8, ferner umfassend das neue Infusionsgerät und das aktive Infusionsgerät, wobei die Signale zwischen dem neuen und dem aktiven Infusionsgerät übertragen werden und wobei die Einstellungen, die aktuell von dem aktiven Infusionsgerät bei der laufenden Infusion des Medikaments an den Patienten verwendet werden, automatisch von einem Speicher des aktiven Infusionsgeräts in einen Speicher des neuen Infusionsgeräts übertragen werden.

10. System nach einem der Ansprüche 1 bis 9, wobei der eine oder die mehreren Prozessoren einen Server umfassen, der entfernt von dem neuen und dem aktiven Infusionsgerät angeordnet ist, wobei das System ferner umfasst:
eine Lokalisierungsvorrichtung, die konfiguriert ist zum Identifizieren des neuen und des aktiven Infusionsgeräts im vordefinierten Bereich und zum Bestimmen der Orientierung des neuen Infusionsgeräts.

11. Nichtflüchtiger maschinenlesbarer Datenträger mit darauf gespeicherten Anweisungen, die, wenn von einer elektronischen Vorrichtung ausgeführt, die elektronische Vorrichtung veranlassen, ein Verfahren durchzuführen, umfassend:
Identifizieren, dass ein neues Infusionsgerät in einen vordefinierten Bereich bewegt wurde, der einem aktiven Infusionsgerät zugeordnet ist, basierend auf Signalen, die zwischen dem neuen und dem aktiven Infusionsgerät übertragen werden, wobei das aktive Infusionsgerät aktuell eine laufende Infusion eines Medikaments an einen Patienten durchführt;
Bestimmen, responsiv auf das Identifizieren des neuen Infusionsgeräts im vordefinierten Bereich mit dem aktiven Infusionsgerät, dass eine Orientierung des neuen Infusionsgeräts darauf hinweist, dass es zum Ersetzen oder Ergänzen des aktiven Infusionsgeräts eingesetzt wird;
responsiv auf das Bestimmen der Orientierung, automatisch das neue Infusionsgerät mit Einstellungen zu programmieren, die aktuell von dem aktiven Infusionsgerät bei der laufenden Infusion des Medikaments an den Patienten verwendet werden, ohne Benutzereingabe der Einstellungen; und
Aktivieren des neuen Infusionsgeräts zum Initiieren einer neuen Infusion oder zum Fortsetzen der laufenden Infusion des Medikaments basierend auf den automatisch programmierten Einstellungen.

12. Nichtflüchtiger maschinenlesbarer Datenträger nach Anspruch 11, wobei der vordefinierte Bereich mindestens Teil eines neuen Versorgungsbereichs ist, der sich von einem Versorgungsbereich unterscheidet, in dem das neue Infusionsgerät zuletzt programmiert wurde, wobei das Verfahren ferner umfasst:
Identifizieren des neuen Versorgungsbereichs für das neue Infusionsgerät;
Bestimmen von Infusionspumpenparametern, die dem neuen Versorgungsbereich zugeordnet sind, wobei die Infusionspumpenparameter einen oder mehrere harte oder weiche Grenzwerte für eine oder mehrere Infusionseinstellungen umfassen, die den Einstellungen entsprechen, die aktuell von dem aktiven Infusionsgerät bei der laufenden Infusion des Medikaments an den Patienten verwendet werden; und
automatisches Programmieren des neuen Infusionsgeräts mit den Infusionspumpenparametern, die dem neuen Versorgungsbereich zugeordnet sind, responsiv auf das Bestimmen der Orientierung.

## Revendications

1. Un système comprenant :
un ou plusieurs processeurs configurés pour :
identifier qu'un nouveau dispositif d'infusion s'est déplacé dans une zone prédéterminée associée à un dispositif d'infusion actif sur la base de signaux transmis entre le nouveau dispositif d'infusion et le dispositif d'infusion actif, le dispositif d'infusion actif effectuant actuellement une infusion en cours d'un médicament à un patient ;
déterminer, en réponse à l'identification du nouveau dispositif d'infusion se déplaçant dans la zone prédéterminée avec le dispositif d'infusion actif, qu'une orientation du nouveau dispositif d'infusion indique que le nouveau dispositif d'infusion arrive pour remplacer ou compléter le dispositif d'infusion actif ;
en réponse à la détermination de l'orientation, programmer automatiquement le nouveau dispositif d'infusion avec des paramètres actuellement utilisés par le dispositif d'infusion actif dans l'infusion en cours du médicament au patient, sans saisie manuelle desdits paramètres par l'utilisateur ; et
activer le nouveau dispositif d'infusion pour initier une nouvelle infusion ou poursuivre l'infusion en cours du médicament sur la base des paramètres programmés automatiquement.

2. Le système selon la revendication 1, dans lequel la zone prédéterminée constitue au moins une partie d'une nouvelle zone de soins différente d'une zone de soins dans laquelle le nouveau dispositif d'infusion a été programmé en dernier lieu, dans lequel le ou les processeurs sont en outre configurés pour :
identifier la nouvelle zone de soins pour le nouveau dispositif d'infusion ;
déterminer des paramètres de pompe d'infusion associés à la nouvelle zone de soins ; et
programmer automatiquement le nouveau dispositif d'infusion avec les paramètres de pompe d'infusion associés à la nouvelle zone de soins en réponse à la détermination de l'orientation.

3. Le système selon la revendication 2, dans lequel les paramètres de pompe d'infusion comprennent une ou plusieurs limites dures ou souples appliquées à un ou plusieurs réglages d'infusion correspondant aux réglages actuellement utilisés par le dispositif d'infusion actif dans l'infusion en cours du médicament au patient.

4. Le système selon la revendication 2 ou 3, dans lequel le ou les processeurs sont en outre configurés pour déterminer que le nouveau dispositif d'infusion est autorisé pour la nouvelle zone de soins avant de programmer automatiquement le nouveau dispositif d'infusion.

5. Le système selon l'une quelconque des revendications 2 à 4, dans lequel le ou les processeurs sont en outre configurés pour :
déterminer qu'un ou plusieurs paramètres associés au nouveau dispositif d'infusion sont soumis à restriction d'utilisation dans la nouvelle zone de soins ;
fournir, pour affichage sur le nouveau dispositif d'infusion, une demande de confirmation de levée de la restriction ;
recevoir une confirmation de levée de la restriction ; et
programmer, en réponse à la réception de la confirmation, le nouveau dispositif d'infusion pour administrer le médicament dans la nouvelle zone de soins selon le ou les paramètres sans la restriction.

6. Le système selon l'une quelconque des revendications 1 à 5, dans lequel la détermination que l'orientation du nouveau dispositif d'infusion indique que le nouveau dispositif d'infusion arrive pour remplacer ou compléter le dispositif d'infusion actif comprend :
la détection d'une distance entre le nouveau dispositif d'infusion et le dispositif d'infusion actif, et d'un mouvement du nouveau dispositif d'infusion par rapport au dispositif d'infusion actif ; et
la mise en correspondance de la distance et du mouvement avec un modèle prédéterminé associé à une intention de remplacer ou de compléter un dispositif d'infusion par un autre,
dans lequel le nouveau dispositif d'infusion est programmé automatiquement en outre en réponse à ladite correspondance.

7. Le système selon la revendication 6, dans lequel le ou les processeurs sont en outre configurés pour identifier un troisième dispositif d'infusion dans la zone prédéterminée, le troisième dispositif d'infusion effectuant actuellement une infusion en cours d'un médicament à un patient respectif, la mise en correspondance de la distance et du mouvement comprenant :
déterminer que la distance et le mouvement correspondent davantage au remplacement ou au complément du dispositif d'infusion actif qu'au troisième dispositif d'infusion.

8. Le système selon la revendication 6 ou 7, dans lequel le ou les processeurs sont en outre configurés pour :
fournir, pour affichage sur un écran du nouveau dispositif d'infusion, une interface utilisateur en réponse à la mise en correspondance de la distance et du mouvement avec le modèle prédéterminé, l'interface utilisateur étant préremplie avec un identifiant de patient et demandant une confirmation pour programmer le nouveau dispositif d'infusion avec des paramètres pour le patient, dans lequel, lorsque la distance et le mouvement ne correspondent pas, l'interface utilisateur est accessible mais n'est pas préremplie avec l'identifiant de patient et ne demande pas de confirmation.

9. Le système selon l'une quelconque des revendications 1 à 8, comprenant en outre le nouveau dispositif d'infusion et le dispositif d'infusion actif, dans lequel les signaux sont transmis entre le nouveau dispositif d'infusion et le dispositif d'infusion actif, et dans lequel les paramètres actuellement utilisés par le dispositif d'infusion actif dans l'infusion en cours du médicament au patient sont automatiquement transférés d'une mémoire du dispositif d'infusion actif vers une mémoire du nouveau dispositif d'infusion.

10. Le système selon l'une quelconque des revendications 1 à 9, dans lequel le ou les processeurs comprennent un serveur situé à distance du nouveau dispositif d'infusion et du dispositif d'infusion actif, le système comprenant en outre :
un dispositif de localisation configuré pour identifier le nouveau dispositif d'infusion et le dispositif d'infusion actif dans la zone prédéterminée et configuré pour déterminer l'orientation du nouveau dispositif d'infusion.

11. Un support non transitoire lisible par ordinateur comportant des instructions stockées qui, lorsqu'elles sont exécutées par un dispositif électronique, amènent le dispositif électronique à exécuter un procédé comprenant :
identifier qu'un nouveau dispositif d'infusion s'est déplacé dans une zone prédéterminée associée à un dispositif d'infusion actif sur la base de signaux transmis entre le nouveau dispositif d'infusion et le dispositif d'infusion actif, le dispositif d'infusion actif effectuant actuellement une infusion en cours d'un médicament à un patient ;
déterminer, en réponse à l'identification du nouveau dispositif d'infusion se déplaçant dans la zone prédéterminée avec le dispositif d'infusion actif, qu'une orientation du nouveau dispositif d'infusion indique que le nouveau dispositif d'infusion arrive pour remplacer ou compléter le dispositif d'infusion actif ;
en réponse à la détermination de l'orientation, programmer automatiquement le nouveau dispositif d'infusion avec des paramètres actuellement utilisés par le dispositif d'infusion actif dans l'infusion en cours du médicament au patient, sans saisie manuelle desdits paramètres par l'utilisateur ; et
activer le nouveau dispositif d'infusion pour initier une nouvelle infusion ou poursuivre l'infusion en cours du médicament sur la base des paramètres programmés automatiquement.

12. Le support non transitoire lisible par ordinateur selon la revendication 11, dans lequel la zone prédéterminée constitue au moins une partie d'une nouvelle zone de soins différente d'une zone de soins dans laquelle le nouveau dispositif d'infusion a été programmé en dernier lieu, dans lequel le procédé comprend en outre :
identifier la nouvelle zone de soins pour le nouveau dispositif d'infusion ;
déterminer des paramètres de pompe d'infusion associés à la nouvelle zone de soins, lesdits paramètres comprenant une ou plusieurs limites dures ou souples appliquées à un ou plusieurs réglages d'infusion correspondant aux réglages actuellement utilisés par le dispositif d'infusion actif dans l'infusion en cours du médicament au patient ; et
programmer automatiquement le nouveau dispositif d'infusion avec les paramètres de pompe d'infusion associés à la nouvelle zone de soins en réponse à la détermination de l'orientation.
